# EUROPÄISCHE PATENTSCHRIFT

(11) **EP 1 907 370 B1**
(45) Veröffentlichungstag und Bekanntmachung des Hinweises auf die Patenterteilung: **25.02.2009**
(21) Anmeldenummer: 06776212.0
(22) Anmeldetag: 13.07.2006
(51) Int. Cl.: C07D 281/10, A61K 31/554, A61P 3/06

(54) **NEUES 1,4-BENZOTHIAZEPIN-1,1-DIOXIDDERIVAT MIT VERBESSERTEN EIGENSCHAFTEN, VERFAHREN ZU DESSEN HERSTELLUNG, DIESE VERBINDUNG ENTHALTENDE ARZNEIMITTEL UND DESSEN VERWENDUNG**
NOVEL 1,4-BENZOTHIAZEPINE-1,1-DIOXIDE DERIVATIVE HAVING IMPROVED PROPERTIES, METHOD FOR THE PRODUCTION THEREOF, MEDICAMENTS CONTAINING SAID COMPOUND, AND USE THEREOF
NOUVEAU DERIVE DE 1,4-BENZOTHIAZEPINE-1,1-DIOXYDE PRESENTANT DES PROPRIETES AMELIOREES, PROCEDE DE FABRICATION, MEDICAMENTS CONTENANT CE COMPOSE ET UTILISATION

(30) Priorität: 15.07.2005 DE 102005033100
(43) Veröffentlichungstag der Anmeldung: 09.04.2008
(73) Patentinhaber: Sanofi-Aventis Deutschland GmbH, 65929 Frankfurt am Main (DE)
(72) Erfinder: FRICK, Wendelin, 65926 Frankfurt am Main (DE); GLOMBIK, Heiner, 65926 Frankfurt am Main (DE); HEUER, Hubert, 65926 Frankfurt am Main (DE); SCHAEFER, Hans-Ludwig, 65926 Frankfurt am Main (DE); THEIS, Stefan, 65926 Frankfurt am Main (DE)
(86) Internationale Anmeldenummer: PCT/EP2006/006849
(87) Internationale Veröffentlichungsnummer: WO 2007/009656

(56) Entgegenhaltungen:
- WO-A-20/04085404
- WO-A2-00/61568
- US-A- 6 107 494
- US-A1- 2003 017 996

## Beschreibung

Die Erfindung betrifft ein substituiertes 1,4-Benzothiazepin-1,1-Dioxidderivat und dessen physiologisch verträgliche Salze.

Es sind bereits 1,4-Benzothiazepin-1,1-Dioxidderivate sowie deren Verwendung zur Behandlung von Hyperlipidämie sowie Arteriosklerose und Hypercholesterinämie beschrieben worden (EP 1 169 313).

Der Erfindung lag die Aufgabe zugrunde, eine Verbindung zur Verfügung zu stellen, die im Gegensatz zu den in EP 1 169 313 beschriebenen Verbindungen eine deutlich verbesserte Wirkung zeigt.

Die Erfindung betrifft daher die Verbindung der Formel A 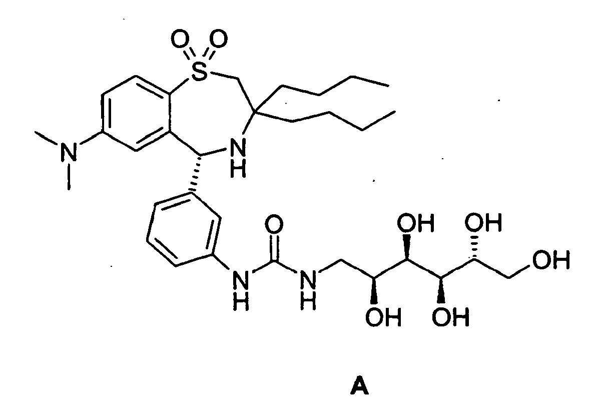 sowie deren pharmazeutisch verträgliche Salze.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindung sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Die erfindungsgemäße Verbindung kann auch in verschiedenen polymorphen Formen vorliegen, z.B. als amorphe und kristalline polymorphe Form. Alle polymorphen Formen der erfindungsgemäßen Verbindung gehören in den Rahmen der Erfindung und sind ein weiterer Aspekt der Erfindung.

Nachfolgend beziehen sich alle Verweise auf "Verbindung(en) gemäß Formel A" auf die Verbindung der Formel A wie vorstehend beschrieben, sowie ihre Salze und Solvate.

Die Verbindung der Formel A kann auch in Kombination mit weiteren Wirkstoffen verabreicht werden.

Die Menge einer Verbindung gemäß Formel A, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,01 mg bis 100 mg (typischerweise von 0,05 mg und 50 mg) pro Tag pro Kilogramm Körpergewicht, z.B. 0,1-10 mg/kg/Tag.

Oral verabreichbare Einzeldosisformulierungen, wie zum Beispiel Tabletten oder Kapseln, können beispielsweise von 1,0 bis 1000 mg, typischerweise von 10 bis 600 mg enthalten. Zur Therapie der oben genannten Zustände können die Verbindungen gemäß Formel A selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel A. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale und perorale (z.B. sublinguale) Verabreichung geeignet sind, wenn gleich die geeignetste Verabreichungsweise in jedem Einzelfall von der Art und Schwere des zu behandelnden Zustandes und von der Art der jeweils verwendeten Verbindung gemäß Formel A abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Poylvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel A enthalten; als Pulver oder Granulate; als Lösung oder Suspension in einer wässrigen oder nicht-wässrigen Flüssigkeit; oder als eine Ölin-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteter Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel A mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Eine weitere Ausführungsform der erfindungsgemäßen pharmazeutischen Zusammensetzungen enthält geeignete Metallsalze, wie zum Beispiel Calcium-, Aluminium-, Eisen-, Kupfer-, Zink-, Magnesium-, Mangan-, oder Zinksalze. Bevorzugt sind Calcium- und Zinksalze, wie zum Beispiel Calciumphosphat, Calciumlactat, Calciumcarbonat, Calciumgluconat, Calciumacetat, Zinkphosphat, Zinklactat, Zinkcarbonat, Zinkgluconat oder Zinkacetat. Der Zusatz dieser Salze zur pharmazeutischen Zusammensetzung kann das Auftreten von Diarrhoe beim Patienten mildern oder verhindern.

### Als weitere Wirkstoffe für die Kombinationspräparate sind geeignet:

Alle Antidiabetika, die in der Roten Liste 2005, Kapitel 12 genannt sind. Sie können mit den erfindungsgemäßen Verbindungen der Formel A insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.

Antidiabetika umfassen Insulin und Insulinderivate, wie z.B. Lantus^{®} (siehe www.lantus.com) oder HMR 1964, schnell wirkende Insuline (siehe US 6,221,633), GLP-1-Derivate wie z.B. diejenigen die in WO 98/08871 von Novo Nordisk A/S, in WO 01/04156 von Zealand oder in WO 00/34331 von Beaufour-Ipsen offenbart wurden, sowie oral wirksame hypoglykämische Wirkstoffe.

Die oral wirksamen hypoglykämischen Wirkstoffe umfassen vorzugsweise Sulphonylfharnstoffe, Biguanidine, Meglitinide, Oxadiazolidindione, Thiazolidindione, Glukosidase-Inhibitoren, Hemmstoffe der Glykogenphosphorylase, Glukagon-Antagonisten, GLP-1-Agonisten, Kaliumkanalöffner, wie z.B. diejenigen, die in WO 97/26265 und WO 99/03861 von Novo Nordisk A/S offenbart wurden, Insulin-Sensitizer, Inhibitoren von Leberenzymen, die an der Stimulation der Glukoneogenese und/oder Glykogenolyse beteiligt sind, Modulatoren der Glukoseaufnahme, des Glukosetransports und der Glukoserückresorption, den Fettstoffwechsel verändernde Verbindungen wie antihyperlipidämische Wirkstoffe und antilipidämische Wirkstoffe, Verbindungen, die die Nahrungsmitteleinnahme verringern, PPAR- und PXR-Agonisten und Wirkstoffe, die auf den ATP-abhängigen Kaliumkanal der Betazellen wirken.

Bei einer Ausführungsform der Erfindung werden die Verbindungen der Formel A in Kombination mit einem HMGCoA-Reduktase Inhibitor wie Simvastatin, Fluvastatin, Pravastatin, Lovastatin, Atorvastatin, Cerivastatin, Rosuvastatin verabreicht.

Bei einer Ausführungsform der Erfindung werden die Verbindungen der Formel I in Kombination mit einem Cholesterinresorptionsinhibitor, wie z.B. Ezetimibe, Tiqueside, Pamaqueside, oder mit einer Verbindung, wie in PCT/EP 2004/00269, PCT/EP 2003/05815, PCT/EP 2003/05814, PCT/EP 2003/05816, EP 0114531 oder US 6,498,156 beschrieben, verabreicht.

Bei einer Ausführungsform der Erfindung werden die Verbindungen der Formel A in Kombination mit einem PPAR gamma Agonist, wie z.B. Rosiglitazon, Pioglitazon, JTT-501, GI 262570, verabreicht.

Bei einer Ausführungsform der Erfindung werden die Verbindungen der Formel A in Kombination mit PPAR alpha Agonist, wie z.B. GW 9578, GW 7647, verabreicht.

Bei einer Ausführungsform der Erfindung werden die Verbindungen der Formel A in Kombination mit einem gemischten PPAR alpha/gamma Agonisten, wie z.B. GW 1536, AVE 8042, AVE 8134, AVE 0847, oder wie in PCT/US 00/11833, PCT/US 00/11490, WO 03/020269 beschrieben verabreicht.

Bei einer Ausführungsform der Erfindung werden die Verbindungen der Formel A in Kombination mit einem Fibrat, wie z.B. Fenofibrat, Clofibrat, Bezafibrat, verabreicht.

Bei einer Ausführungsform der Erfindung werden die Verbindungen der Formel A in Kombination mit einem MTP-Inhibitor, wie z.B. Implitapide, BMS-201038, R-103757, verabreicht.

Bei einer Ausführungsform der Erfindung werden die Verbindungen der Formel A in Kombination mit einem CETP-Inhibitor, wie z.B. JTT-705, verabreicht.

Bei einer Ausführungsform der Erfindung werden die Verbindungen der Formel A in Kombination mit einem polymeren Gallensäureadsorber, wie z.B. Cholestyramin, Colesevelam, verabreicht.

Bei einer Ausführungsform der Erfindung werden die Verbindungen der Formel A in Kombination mit einem LDL-Rezeptorinducer (siehe US 6,342,512) verabreicht.

Bei einer Ausführungsform der Erfindung werden die Verbindungen der Formel A in Kombination mit einem ACAT-Inhibitor, wie z.B. Avasimibe, verabreicht.

Bei einer Ausführungsform der Erfindung werden die Verbindungen der Formel A in Kombination mit einem Antioxidans, wie z.B. OPC-14117, verabreicht.

Bei einer Ausführungsform der Erfindung werden die Verbindungen der Formel A in Kombination mit einem Lipoprotein-Lipase Inhibitor, wie z.B. NO-1886, verabreicht.

Bei einer Ausführungsform der Erfindung werden die Verbindungen der Formel A in Kombination mit einem ATP-Citrat-Lyase Inhibitor, wie z.B. SB-204990, verabreicht.

Bei einer Ausführungsform der Erfindung werden die Verbindungen der Formel A in Kombination mit einem Squalen Synthetase Inhibitor, wie z.B. BMS-188494, verabreicht.

Bei einer Ausführungsform der Erfindung werden die Verbindungen der Formel A in Kombination mit einem Lipoprotein(a) antagonist, wie z.B. CI-1027 oder Nicotinsäure, verabreicht.

Bei einer Ausführungsform der Erfindung werden die Verbindungen der Formel A in Kombination mit einem Lipase Inhibitor, wie z.B. Orlistat, verabreicht.

Bei einer Ausführungsform der Erfindung werden die Verbindungen der Formel A in Kombination mit Insulin verabreicht.

Bei einer Ausführungsform werden die Verbindungen der Formel A in Kombination mit einem Sulphonylharnstoff, wie z.B. Tolbutamid, Glibenclamid, Glipizid oder Glimepirid verabreicht.

Bei einer Ausführungsform werden die Verbindungen der Formel A in Kombination mit einem Biguanid, wie z.B. Metformin, verabreicht.

Bei wieder einer Ausführungsform werden die Verbindungen der Formel A in Kombination mit einem Meglitinid, wie z.B. Repaglinid, verabreicht.

Bei einer Ausführungsform werden die Verbindungen der Formel A in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei einer Ausführungsform werden die Verbindungen der Formel A in Kombination mit einem α-Glukosidase-Inhibitor, wie z.B. Miglitol oder Acarbose, verabreicht.

Bei einer Ausführungsform werden die Verbindungen der Formel I in Kombination mit einem Adenosin A1 Agonisten, wie z. B. solchen, die in EP 0912520 oder PCT/EP06749 beschrieben sind, verabreicht.

Bei einer Ausführungsform werden die Verbindungen der Formel A in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausführungsform werden die Verbindungen der Formel A in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht.

Bei einer weiteren Ausführungsform werden die Verbindungen der Formel A in Kombination mit CART-Modulatoren (siehe "Cocaine-amphetamine-regulated transcript influences energy metabolism, anxiety and gastric emptying in mice" Asakawa, A, et al., M.: Hormone and Metabolic Research (2001), 33(9), 554-558), NPY-Antagonisten z.B. Naphthalin-1-sulfonsäure-{4-[(4-amino-quinazolin-2-ylamino)-methyl]-cyclohexylmethyl}-amid Hydrochlorid (CGP 71683A)), Cannabinoid Rezeptor 1 Antagonisten (siehe z.B. EP 0656354, WO 00/15609 oder WO 02/076949), MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid; (WO 01/91752)), Orexin-Antagonisten (z.B. 1-(2-Methyl-benzoxazol-6-yl)-3-[1,5]naphthyridin-4-yl-harnstoff Hydrochlorid (SB-334867-A)), H3-Agonisten (3-Cyclohexyl-1-(4,4-dimethyl-1,4,6,7-tetrahydro-imidazo[4,5-c]pyridin-5-yl)-propan-1-on Oxalsäuresalz (WO 00/63208)); TNF-Agonisten, CRF-Antagonisten (z.B. [2-Methyl-9-(2,4,6-trimethyl-phenyl)-9H-1,3,9-triaza-fluoren-4-yl]-dipropyl-amin (WO 00/66585)), CRF BP-Antagonisten (z.B. Urocortin), Urocortin-Agonisten, β3-Agonisten (z.B. 1-(4-Chloro-3-methanesulfonylmethyl-phenyl)-2-[2-(2,3-dimethyl-1H-indol-6-yloxy)-ethylamino]-ethanol Hydrochlorid (WO 01/83451)), MSH (Melanocytstimulierendes Hormon)-Agonisten, MCH (melanin-konzentrierendes Hormon) Rezeptor Antagonisten (siehe z.B. WO 03/15769), CCK-A Agonisten (z.B. {2-[4-(4-Chloro-2,5-dimethoxy-phenyl)-5-(2-cyclohexyl-ethyl)-thiazol-2-ylcarbamoyl]-5,7-dimethyl-indol-1-yl}-essigsäure Trifluoressigsäuresalz (WO 99/15525) oder SR-146131 (WO 0244150) oder SSR-125180), Serotonin-Wiederaufnahme-Inhibitoren (z.B. Dexfenfluramine), gemischte Sertonin- und noradrenerge Verbindungen (z.B. WO 00/71549), 5HT-Agonisten z.B. 1-(3-Ethyl-benzofuran-7-yl)-piperazin Oxalsäuresalz (WO 01/09111), Bombesin-Agonisten, Galanin-Antagonisten, Wachstumshormon (z.B. humanes Wachstumshormon), Wachstumshormon freisetzende Verbindungen (6-Benzyloxy-1-(2-diisopropylamino-ethylcarbamoyl)-3,4-dihydro-1H-isochinolin-2-carbonsäuretertiärbutylester (WO 01/85695)), TRH-Agonisten (siehe z.B. EP 0 462 884) entkoppelnde Protein 2- oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881), DA-Agonisten (Bromocriptin, Doprexin), Lipase/Amylase-Inhibitoren (z.B. WO 00/40569), PPAR-Modulatoren (z.B. WO 00/78312), Hemmstoffen der 11β-HSD1 (11-beta-Hydroxysteroiddehydrogenase Typ1) (siehe z.B. WO 01/90094 oder T.Barf et al., J. Med. Chem. (2002), 45, 3813-3815), Hemmstoffen der Acetyl-CoA Carboxylase (ACC; siehe z.B. WO 99/46262), Hemmstoffen der Dipeptidylpeptidase IV (DPP-IV; siehe z.B. EP 1259246), )RXR-Modulatoren oder TR-β-Agonisten verabreicht.

Bei einer Ausführungsform der Erfindung ist der weitere Wirkstoff Leptin; siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform ist der weitere Wirkstoff Dexamphetamin oder - Amphetamin.

Bei einer Ausführungsform ist der weitere Wirkstoff Fenfluramin oder Dexfenfluramin.

Bei noch einer Ausführungsform ist der weitere Wirkstoff Sibutramin.

Bei einer Ausführungsform ist der weitere Wirkstoff Orlistat.

Bei einer Ausführungsform ist der weitere Wirkstoff Mazindol oder Phentermin.

Bei einer Ausführungsform werden die Verbindungen der Formel A in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties &Food Ingredients GmbH, Industriepark Höchst, 65926 Frankfurt / Main)) verabreicht. Die Kombination mit Caromax^{®} kann in einer Zubereitung erfolgen, oder durch getrennte Gabe von Verbindungen der Formel A und Caromax^{®}. Caromax^{®} kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

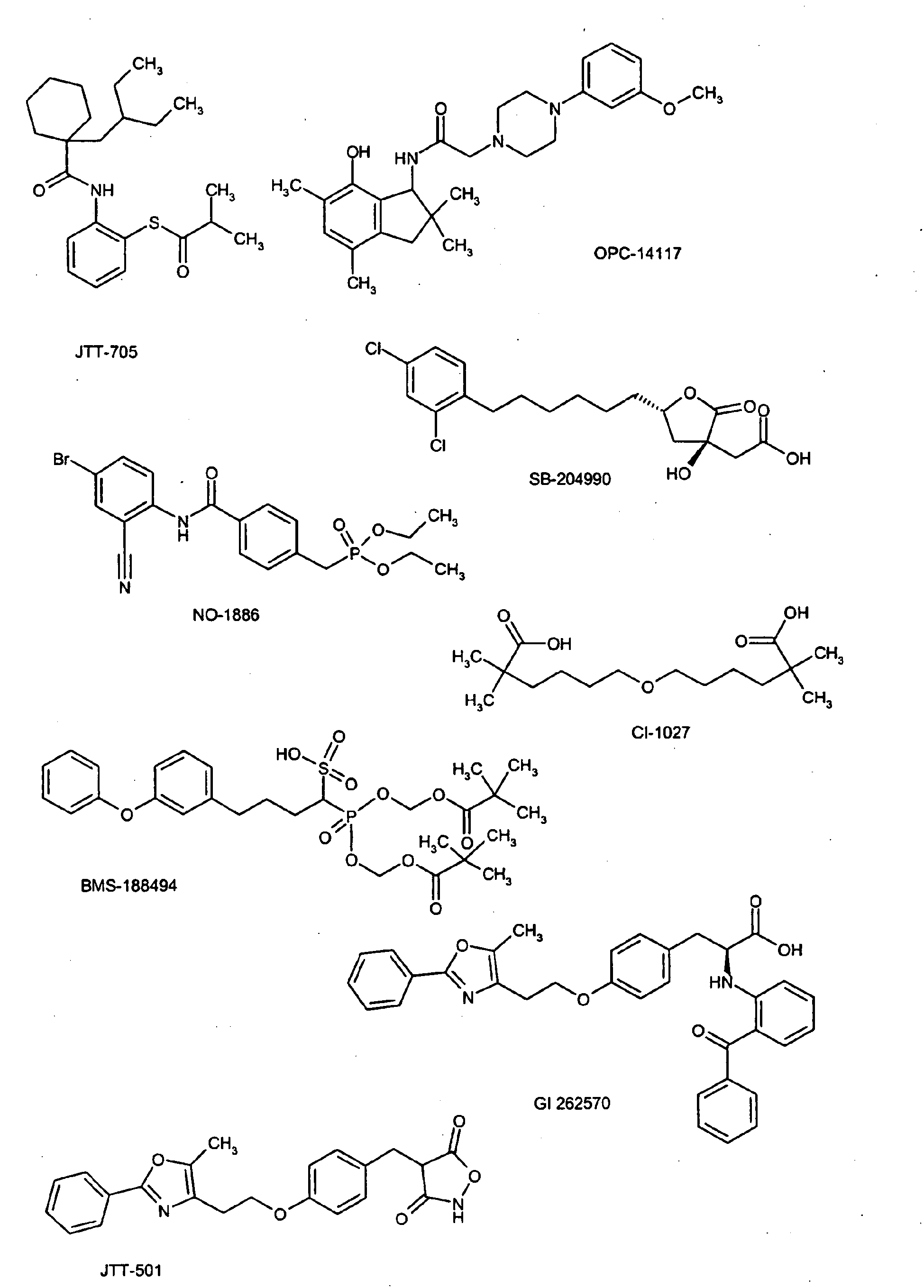

### Beispiel A

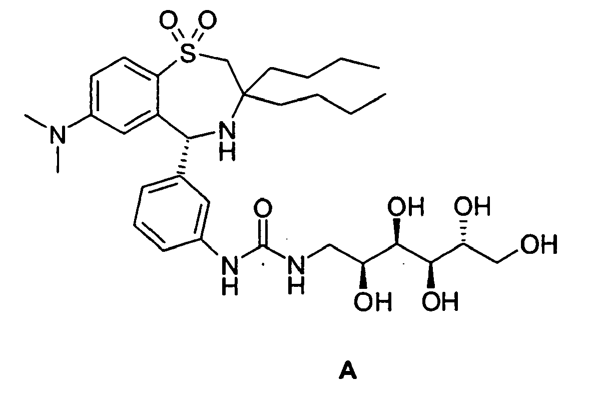

Die Verbindung A wurde wie folgt hergestellt:

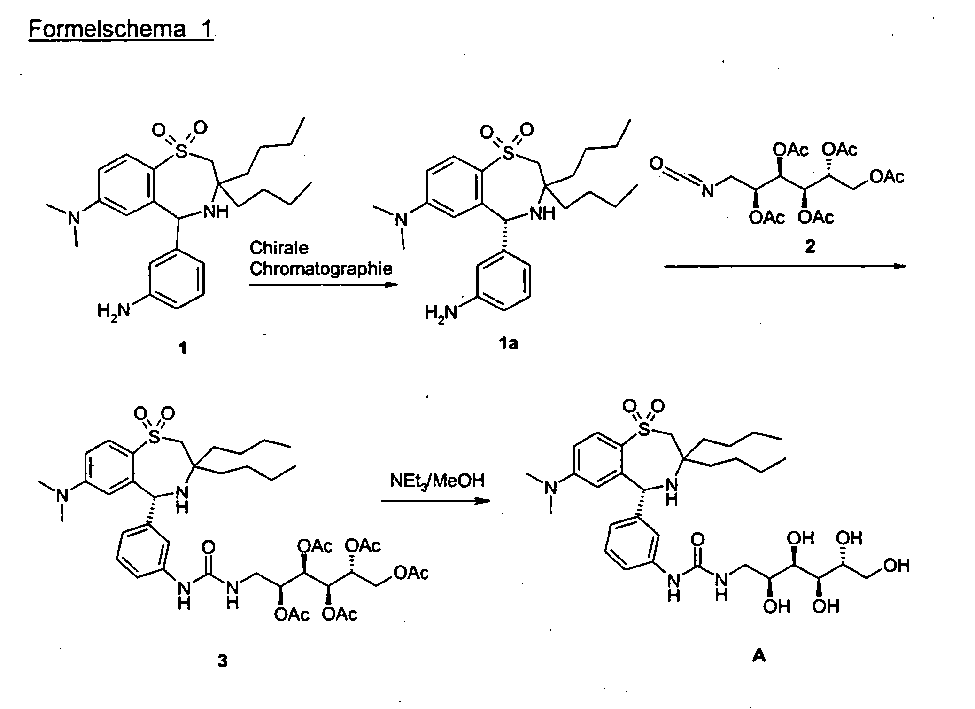

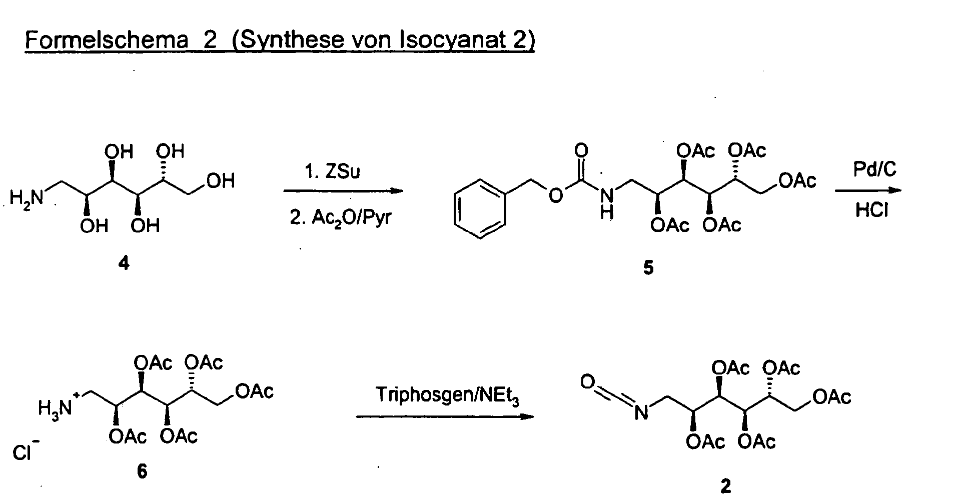

### Synthese von Verbindung 1a:

Die enatiomerenreine Verbindung **1a** wird aus dem racemischen Anilid 1 (EP 1 169 313 Verbindung **8a/b**) durch eine chirale Chromatographie mit Chrialpak AS-H76 mit dem Eluent n-Heptan/Methanol/Ethanol 10/1/1 plus 0. 1 % DEA erhalten. Durch Kristallisation aus THF/EE/n-Heptan kann die Verbindung **1a** hochgereinigt werden (ee von 99.9%). Das Eutomer hat einen positiven Drehwert und die absolute Stereochemie wurde mit einer Einkristallröntgenstruktur bestimmt.

### Synthese von Verbindung 5:

4,5 g Glucosamin **4** (Aldrich) werden in 100 ml DMF bei 80 °C gelöst und dann gibt man 5 g N-(Benzyloxycarbonyloxy)-succinimid (ABCR) zu. Nach 20 Minuten tropft man 100 ml Essigsäureanhydrid/Pyridin (1: 1) zu und hält die Reaktionslösung 1 Stunde bei 50 °C. Das Lösungsmittel wird am Hochvakuum abdestilliert und der Rückstand mit Flashchromatographie gereinigt. Ausbeute 10.1 g (77%) 5 als farbloser Feststoff. DC (n- Heptan/ Ethylacetat 1:2). R_{f} = 0.4.
C₂₄H₃₁NO₁₂ (525.51). MS (M+H)⁺ = 525.25.

### Synthese von Verbindung 6:

17,45 g Pentaacetat **5** werden in 200 ml 0,25 M methanolischer Salzsäure gelöst und unter Argon werden 1,3 g 10 % Palladium auf Aktivkohle zugegeben. Bei einem Wasserstoffdruck von 5 bar hydriert man eine Stunde bei Raumtemperatur. Der Katalysator wird abgetrennt und rund 150 ml Methanol abdestilliert. Nach Zugabe von MTB-Ether fällt das Hydrochlorid **6** aus. Man erhält 13 g (92%) farbloser Feststoff **6**. C₁₆H₂₅NO₁₀xHCl (427,84), MS (M+H)⁺ = 392,21.

### Synthese von Verbindung 2:

1,5 g Hydrochlorid **6** werden in 50 ml Methylenchlorid suspendiert und unter Argon auf 0°C gekühlt. Zu dieser Suspension gibt man nacheinander 1.5 g Triphosgen und 3 ml Triethylamin und rührt 1 Stunde bei 0°C. Die Reaktionslösung wird mit 50 ml n-Heptan/Ethylacetat (1:1) verdünnt und über wenig Kieselgel filtriert. Das Lösungsmittel wird abdestilliert und man erhält 1,52 g amorpher Feststoff **2** als Rohprodukt das ohne weitere Reinigung direkt in die nächste Stufe eingesetzt wird.

### Synthese von Verbindung 3:

5.45 g (12,3 mmol) Anilid **1a** und 3.58 g (8,5 mmol) Isocyanat **2** werden in 150 ml Methylenchlorid bei Raumtemperatur gelöst. Nach einer Stunde bei Raumtemperatur wird eingeengt und der Rückstand mit Flashchromatographie gereinigt. Ausbeute 4.72 g (64%) **3** als farbloser Feststoff. DC (n- Heptan/ Ethylacetat 1:2). R_{f} = 0.3. C₄₂H₆₀N₄O₁₃S (861,03). MS (M+H)⁺ = 861,59.

### Synthese von Verbindung A:

4.7 g **3** werden in 60 ml Methanol und 20 ml Triethylamin gelöst und 16 Stunden bei Raumtemperatur stehen gelassen. Die Lösung wird eingeengt und der Rückstand mit Flashchromatographie (Methylenchlorid/ Methanol/ konz. Ammoniak 30/10/3) gereinigt. Ausbeute 2.17g (60%) **A** als amorpher Feststoff. DC (Methylenchlorid/ Methanol/ konz. Ammoniak 30/10/3). R_{f}= 0.4. C₃₂H₅₀N₄O₈S (650.84). MS (M + H)⁺ = 651,34.

Die biologische Prüfung der erfindungsmäßen Verbindung A erfolgte durch den in vitro IBAT Hemmtest. Diese Prüfung untersucht die Wirkung der erfindungsmäßen Verbindung auf die Transportaktivität des rekombinant exprimierten humanen natrium-abhängigen ilealen Gallensäuretransporters (IBAT = ileal Na⁺/bile acid cotransporter, ASBT = apical sodium-dependent bile acid transporter, SLC10A2 = solute carrier family 10, member 2).

Vorarbeiten und Durchführung des in vitro IBAT Hemmtestes:

### 1. Klonierung eines Expressionsvektors für den humanen IBAT

Die cDNA (complementary deoxyribonucleic acid) des humanen IBAT wurde über molekularbiologische Standardmethoden, wie z.B. in Molecular Cloning: A Laboratory Manual von Joseph Sambrook und David Russell beschrieben, kloniert und in den pcDNA1 Vektor der Fa. Invitrogen eingebracht. Die anschließende Sequenzierung des Inserts ergab vollständige Identität mit den Basen 599 bis 1645 der von P.A. Dawson beschriebenen und in der GenBank Sequenzdatenbank hinterlegten Basensequenz für den humanen IBAT (GenBank Accesion Nummer: U10417). Die Basen 599 bis 1645 entsprechen der kompletten kodierenden Region des humanen IBAT.

### 2. Herstellung einer rekombinanten Zelllinie mit konstitutiver Expression des humanen IBAT

Der Expressionsvektor für den humanen IBAT wurde mittels stabiler Transfektion in CHO (chinese hamster ovary) Zellen eingeführt. Zur Selektion von Einzelzellklonen wurde dem Zellkulturmedium (Ham's F12 Medium supplementiert mit 10% fötalem Kälberserum, 100 Einheiten/ml Penicillin, 100 Einheiten/ml Streptomycin) 400µg/ml Geneticin zugesetzt. Die Funktionalität der aus der Selektion resultierenden Einzelzellklone wurde über deren Aufnahmeaktivität für radioaktiv markierte Taurocholsäure ([³H]-TCA) getestet. Derjenige Zellklon mit der höchsten Aufnahmeaktivität für [³H]-TCA, nachfolgend CHO-hIBAT bezeichnet, wurde für die weiteren Tests ausgewählt und weiterhin in Anwesenheit von 400µg/ml Geneticin kultiviert.

### 3. Messung der hemmenden Wirkung der erfindungsmäßigen Verbindung auf die IBAT-abhängige Aufnahme von Taurocholsäure in Zellen

CHO-hIBAT Zellen wurden in einer Konzentration von 40 000 Zellen pro Well in Poly-D-Lysin-beschichtete 96-Well Platten in Zellkulturmedium ausgesät und für 24 h kultiviert. Dann wurden die Zellen einmal mit natrium-freiem Transport-Assay-Puffer (140mM Cholinchlorid, 2mM Kaliumchlorid, 1 mM Magnesiumchlorid, 1 mM Kalziumchlorid, 10mM HEPES/Tris, pH7,5) gewaschen und anschließend entweder mit natrium-freiem Transport-Assay-Puffer als Negativkontrolle oder mit natrium-haltigem Transport Assay Puffer (140mM Natriumchlorid, 2mM Kaliumchlorid, 1mM Magnesiumchlorid, 1mM Kalziumchlorid, 10mM HEPES/Tris, pH7,5) als Positivkontrolle für 30 min bei Raumtemperatur inkubiert. Gleichzeitig wurden auch die Testwells in Anwesenheit der zu untersuchenden Verbindung in unterschiedlicher Konzentration für 30 min bei Raumtemperatur in natrium-haltigem Transport-Assay-Puffer inkubiert. Die Testsubstanzen wurden ausgehend von einer 10mM Stammlösung in Dimethylsulfoxid entsprechend in Transport-Assay-Puffer verdünnt (40µl/Well). Der Test wurde anschließend durch Zugabe von 10µl/Well einer Mischung aus radioaktiv markierter Taurocholsäure ([³H]-TCA) und unmarkierter Taurocholsäure gestartet. Die Endkonzentration der Taurocholsäure im Test lag bei 10µM. Nach einer Inkubationszeit von 60 min bei Raumtemperatur wurde die Reaktion durch Zugabe von 100µl/Well natrium-freiem Transport-Assay-Puffer (4°C) gestoppt und jedes Well dreimal mit natrium-freiem Transport-Assay-Puffer gewaschen. Zuletzt wurde jedem Well 100µl Szintillationsflüssigkeit zugegeben und die in die Zellen aufgenommene Radioaktivität in einem MicroBeta Scintillation Microplate Reader der Fa. Wallac bestimmt.

Die halbmaximale Hemmwirkung der Testverbindung (IC50 Wert, inhibitory concentration 50) wurde folgendermaßen bestimmt:
1. Feststellung des Wertes für 0% Inhibition. Dies ist der Messwert bei Abwesenheit von Substanz, gemessen in natrium-haltigem Transport-Assay-Puffer.
2. Feststellung des Wertes für 100% Inhibition. Dies ist der Messwert bei Abwesenheit von Substanz, gemessen in natrium-freiem Transport-Assay-Puffer.
3. Errechnung der prozentualen Hemmwerte derjenigen Messungen, die in Anwesenheit unterschiedlicher Konzentrationen der zu untersuchenden Verbindung durchgeführt wurden. Daraus konnte dann diejenige Konzentration der Verbindung ermittelt werden, welche die Aufnahme der Taurocholsäure um 50% reduziert (IC50 Wert).

### Für Beispiel A ergab sich IC50(human IBAT): 0.155µM

Zum Vergleich wurde die strukturell ähnlichste Verbindung aus EP 1 169 313 ebenfalls gemessen. Dabei ergab sich für die Verbindung 11a (aus Beispiel 5) der Formel 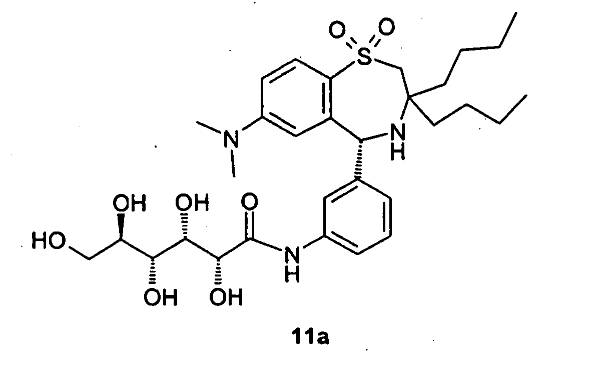 IC50(human IBAT): 0.319µM

Die erfindungengemäße Verbindung A zeigt gegenüber diesem Vergleichsbeispiel aus EP 1 169 313 eine um 206% höhere Wirkung.

## Patentansprüche

1. Verbindung der Formel A, 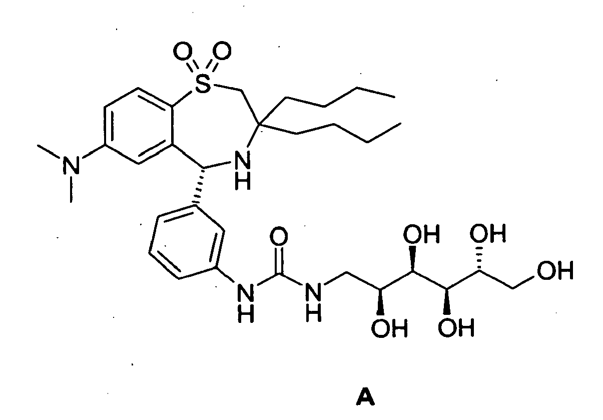 sowie deren pharmazeutisch verträgliche Salze.

2. Arzneimittel enthaltend die Verbindung gemäß Anspruch 1.

3. Arzneimittel enthaltend die Verbindung gemäß Anspruch 1 und mindestens einen weiteren Wirkstoff.

4. Arzneimittel, gemäß Anspruch 3, **dadurch gekennzeichnet, daß** es als weiteren Wirkstoff eine oder mehrere Verbindungen, die den Lipidstoffwechsel normalisieren, enthält.

5. Arzneimittel, gemäß Anspruch 3 oder 4, **dadurch gekennzeichnet, daß** es als weiteren Wirkstoff Simvastatin, Fluvastatin, Pravastatin, Lovastatin, Atorvastatin, Cerivastatin, Rosuvastatin, Fenofibrat, Clofibrat, Bezafibrat, Metformin, Ezetimibe, Tiqueside, Pamaqueside, Tolbutamid, Glibenclamid, Glipizid oder Glimepirid enthält.

6. Arzneimittel, gemäß einem der Ansprüche 2, 3, 4 oder 5, **dadurch gekennzeichnet, daß** es als weiteren Hilfsstoff eine oder mehrere Metallsalze enthält.

7. Verbindung gemäß Anspruch 1 zur Anwendung als Medikament zur Behandlung von Lipidstoffwechselstörungen.

8. Verfahren zur Herstellung eines Arzneimittels enthaltend die Verbindung gemäß Anspruch 1, **dadurch gekennzeichnet, daß** der Wirkstoff mit einem pharmazeutisch geeigneten Träger vermischt wird und diese Mischung in eine für die Verabreichung geeignete Form gebracht wird.

9. Verwendung der Verbindung gemäß Anspruch 1 zur Herstellung eines Medikaments zur Behandlung von Hyperlipidämie.

10. Verwendung der Verbindung gemäß Anspruch 1 zur Herstellung eines Medikaments zur Senkung des Serumcholesterinspiegels.

11. Verwendung der Verbindung gemäß Anspruch 1 zur Herstellung eines Medikaments zur Behandlung arteriosklerotischer Erscheinungen.

12. Verwendung der Verbindung gemäß Anspruch 1 zur Herstellung eines Medikaments zur Behandlung von Insulin Resistenz.

## Claims

1. A compound of the formula A 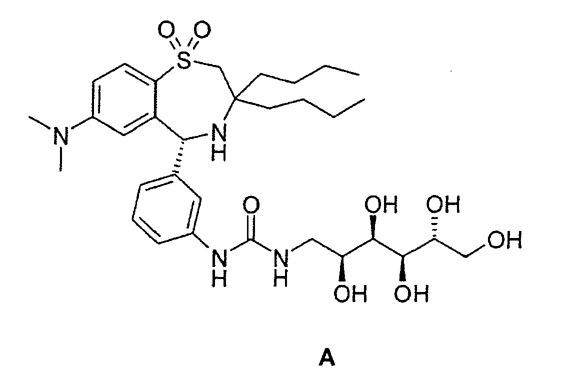 or a pharmaceutically acceptable salt thereof.

2. A medicament, comprising the compound as claimed in claim 1.

3. A medicament, comprising the compound as claimed in claim 1 and at least one further active compound.

4. A medicament as claimed in claim 3, which comprises, as further active compound, one or more compounds which normalize lipid metabolism.

5. A medicament as claimed in claim 3 or 4, which comprises, as further active compound, simvastatin, fluvastatin, pravastatin, lovastatin, atorvastatin, cerivastatin, rosurastatin, fenofibrate, clofibrate, bezafibrate, metformin, ezetimibe, tiqueside, pamaqueside, tolbutamide, glibenclamide, glipizide or glimepiride.

6. A medicament as claimed in any of claims 2, 3, 4 or 5, which comprises, as further auxiliary, one or more metal salts.

7. A compound as claimed in claim 1 for use as a medicament for the treatment of disorders of lipid metabolism.

8. A process for preparing a medicament comprising the compound as claimed in claim 1, which comprises mixing the active compound with a pharmaceutically acceptable carrier and bringing this mixture into a form suitable for administration.

9. The use of the compound as claimed in claim 1 for preparing a medicament for the treatment of hyperlipidemia.

10. The use of the compound as claimed in claim 1 for preparing a medicament for lowering the serum cholesterol concentration.

11. The use of the compound as claimed in claim 1 for preparing a medicament for treating arteriosclerotic symptoms.

12. The use of the compound as claimed in claim 1 for preparing a medicament for treating insulin resistance.

## Revendications

1. Composé de formule A, 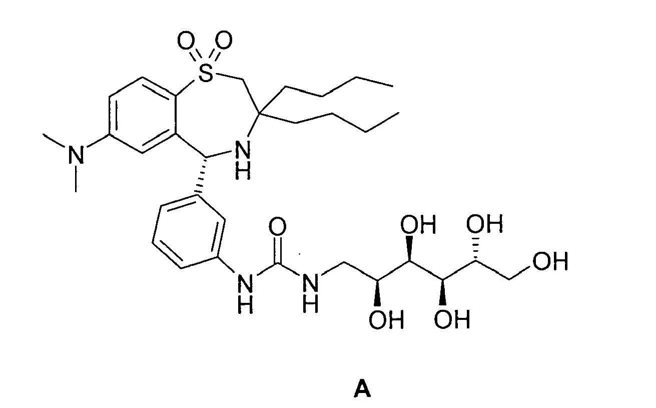 ainsi que ses sels acceptables d'un point de vue pharmaceutique.

2. Médicament contenant le composé selon la revendication 1.

3. Médicament contenant le composé selon la revendication 1 et au moins un autre principe actif.

4. Médicament selon la revendication 3, **caractérisé en ce qu'**il contient en tant qu'autre principe actif un ou plusieurs composés qui normalisent le métabolisme des lipides.

5. Médicament selon la revendication 3 ou 4, **caractérisé en ce qu'**il contient en tant qu'autre principe actif la simvastatine, la fluvastatine, la pravastatine, la lovastatine, l'atorvastatine, la cérivastatine, la rosuvastatine, le fénofibrate, le clofibrate, le bezafibrate, la metformine, l'ézétimibe, le tiquéside, le pamaquéside, le tolbutamide, la glibenclamide, le glipizide ou le glimepiride.

6. Médicament selon l'une des revendications 2, 3, 4 ou 5, **caractérisé en ce qu'**il contient en tant qu'autre adjuvant un ou plusieurs sels métalliques.

7. Composé selon la revendication 1, pour utilisation en tant que médicament destiné au traitement des troubles du métabolisme des lipides.

8. Procédé de préparation d'un médicament contenant le composé selon la revendication 1, **caractérisé en ce que** le principe actif est mélangé à un excipient approprié d'un point de vue pharmaceutique, et ce mélange est mis sous une forme convenant à l'administration.

9. Utilisation du composé selon la revendication 1 pour préparer un médicament destiné au traitement de l'hyperlipidémie.

10. Utilisation du composé selon la revendication 1 pour préparer un médicament destiné à diminuer le taux sérique du cholestérol.

11. Utilisation du composé selon la revendication 1 pour préparer un médicament destiné au traitement des phénomènes artériosclérotiques.

12. Utilisation du composé selon la revendication 1 pour préparer un médicament destiné au traitement de la résistance à l'insuline.
